# FASCICULE DE BREVET EUROPEEN

(11) **EP 2 615 989 B1**
(45) Date de publication et mention de la délivrance du brevet: **14.03.2018**
(21) Numéro de dépôt: 11773105.9
(22) Date de dépôt: 16.09.2011
(51) Int. Cl.: A61B 17/70

(54) **SYSTEME DE PINCEMENT D'EPINEUSES ET SES APPLICATIONS**
SYSTEM ZUM ERGREIFEN VON DORNFORTSÄTZEN UND SEINE VERWENDUNG
SYSTEM FOR GRIPPING SPINOUS PROCESSES, AND USES THEREOF

(30) Priorité: 17.09.2010 FR 1057472
(43) Date de publication de la demande: 24.07.2013
(73) Titulaire: Spineart SA, 1217 Meyrin (CH)
(72) Inventeur: KEIPER, Glenn, Eugene Oregon 97403 (US); LEVIEUX, Jérôme, Laguna Beach, CA 92651 (US)
(74) Mandataire: Cabinet Plasseraud
(86) Numéro de dépôt international: PCT/FR2011/052133
(87) Numéro de publication internationale: WO 2012/035275

(56) Documents cités:
- US-A1- 2006 247 640
- US-A1- 2008 177 391
- US-A1- 2008 183 211
- US-A1- 2008 183 218
- US-A1- 2009 265 006

## Description

La présente invention concerne un système de pincement d'épineuses et ses applications.

Les pathologies rachidiennes sont traitées de différentes manières suivant leurs gravités et leurs particularités. L'une d'entre-elles est la chirurgie. En particulier, l'immobilisation de deux ou plusieurs vertèbres adjacentes est une technique reconnue et efficace. Ce blocage de deux ou plusieurs segments vertébraux aboutit à la fusion de deux ou plusieurs vertèbres entre-elles.

Plusieurs méthodes existent pour « fixer » ces vertèbres, la plus connue étant celle qui consiste à implanter des vis pédiculaires et à les relier avec des barres longitudinales. Une autre méthode consiste à pincer les épineuses des vertèbres concernées afin de bloquer leur mouvement relatif. Pour cela on utilise un dispositif inter-épineux dit "de fusion".

Différents systèmes de ce type existent sur le marché, notamment le système commercialisé par la société Medtronic sous la dénomination SPIRE Plate® ou le système commercialisé par la société Lanx sous la dénomination ASPEN®.

Le système SPIRE Plate fonctionne bien pour pincer l'épineuse, cependant sa mise en place est quelque peu complexe car le chirurgien doit maintenir pincées les deux plaques et en même temps serrer un écrou. Cette manoeuvre nécessite généralement une aide car elle est délicate à réaliser avec seulement deux mains. De plus le système SPIRE Plate ne propose pas de zone dans laquelle le chirurgien peut placer de la greffe osseuse destinée à fusionner les deux épineuses.

Le système ASPEN propose un cylindre dans lequel le chirurgien peut placer la greffe osseuse. Mais ce cylindre est réalisé en titane ou en matériau métallique et ne permet pas une visualisation aisée de la greffe lors des contrôles radiographiques. De plus, comme pour le système SPIRE Plate®, la mise en place est complexe. Le principe est le même et le chirurgien doit donc maintenir la pression sur les épineuses quand il serre la vis de blocage. Pour ce dispositif aussi il convient de se faire aider, la manoeuvre étant plus facile à quatre mains.

US 2008/183211 décrit un implant pour apophyses épineuses. L'implant comprend une entretoise et deux plateaux en prise avec l'entretoise, un fixe et un mobile. Le plateau mobile peut être rapproché du plateau fixe pour stabiliser l'implant. L'implant est fixé en place en bloquant le plateau mobile par serrage d'une vis contre la face postérieure de l'entretoise. Un instrument de vissage est donc nécessaire pour installer l'implant et la mise en oeuvre de l'implant est donc lente et complexe. Il ne faut en particulier pas oublier le tournevis avec l'embout adapté.

Il serait souhaitable de disposer de nouveaux systèmes qui soient plus simples à mettre en place et qui ne nécessitent pas le serrage d'une vis et donc l'utilisation d'une clé ou tournevis pour bloquer le système. Il serait aussi souhaitable de disposer de nouveaux systèmes qui ne nécessitent pas une pointe ou un auxiliaire analogue pour bloquer le système par écartement de deux parties d'une pièce en s'insérant dans entre ces deux parties, notamment en raison du risque de perte de cette pièce.

Il serait aussi souhaitable de permettre au chirurgien d'installer une greffe osseuse.

Il serait aussi souhaitable de permettre de contrôler la greffe osseuse notamment par contrôles radiographiques.

Après de longues recherches la demanderesse a élaboré un nouveau dispositif, donnant toute satisfaction, fondé sur un système autobloquant par crantage. De plus, une chambre permet au chirurgien d'installer une greffe osseuse.

C'est pourquoi la présente demande a pour objet un système de pincement d'épineuses caractérisé en ce qu'il comprend un premier et un second plateau installés face à face et un arbre éventuellement tubulaire installé environ perpendiculairement aux deux plateaux et traversant au moins partiellement l'un d'eux, les plateaux comprenant une face interne et une face externe, en ce qu'une surface de la face interne des plateaux est munie d'aspérités en relief, en ce que le premier plateau est mobile relativement au second plateau, en ce que le premier plateau est fixe en translation par rapport à l'arbre et en ce que l'arbre et le second plateau forment un mécanisme à cliquet anti-retour comprenant une série de crans coopérant avec le mécanisme à cliquet anti-retour pour que le rapprochement des plateaux soit irréversible sans aide extérieure, en conséquence de quoi des épineuses peuvent être efficacement pincées entre les deux plateaux.

Pour rappel, un exemple classique de mécanisme à cliquet anti-retour est la roue à rochet, dispositif autorisant un mécanisme rotatif à tourner dans un seul sens. Une telle roue est munie sur sa circonférence d'encoches provoquant dans le sens choisi le soulèvement d'un cliquet pour lui laisser passage mais bloquant la rotation dans l'autre sens.

Le premier plateau, fixe en translation par rapport à l'arbre, peut être d'une pièce avec l'axe. Toutefois, dans des conditions préférentielles de mise en oeuvre de l'invention, le premier plateau et l'arbre sont deux pièces distinctes. Les épineuses étant de forme aléatoire, il est en effet souhaitable qu'un plateau, de préférence le premier plateau, puisse s'incliner selon un angle par rapport à l'arbre.

Avantageusement, lorsque le premier plateau et l'arbre sont deux pièces distinctes, une des extrémités de l'arbre comprend une lumière circulaire installée diamétralement par rapport à l'axe de l'arbre, par exemple en forme d'anneau.

Le premier plateau peut alors comprendre une cavité pour le passage de cette extrémité et un moyen utilisé comme axe pour la lumière circulaire pour permettre à l'arbre de se mouvoir autour d'un point fixe. Ce moyen peut notamment être une vis, une cheville, un goujon, ou un goujon partiellement fileté. Si son diamètre est plus faible que celui de l'ouverture circulaire radiale, le premier plateau peut se mouvoir de manière limitée dans une direction diamétrale de l'arbre, autour d'un point fixe situé dans l'axe de ce moyen. Ainsi, le premier plateau peut s'incliner selon un angle par rapport à l'arbre et le système de pincement d'épineuses peut s'adapter à des épineuses de formes variées. Le premier plateau et le second plateau ne sont donc pas systématiquement parallèles.

Le second plateau peut se déplacer en translation selon l'axe de l'arbre pour se rapprocher du premier plateau et former une sorte d'étau. Le second plateau comporte un puits permettant le passage de l'arbre et autorisant le rapprochement des plateaux.

L'arbre et le second plateau forment un mécanisme à cliquet anti-retour pour que le rapprochement des plateaux soit irréversible sans aide extérieure. Ce mécanisme empêche le retour en arrière du second plateau lorsque celui-ci se rapproche du premier plateau.

Dans des conditions préférentielles de mise en oeuvre de l'invention, l'arbre est une tige crantée. L'arbre peut avoir toute section transversale et cette section est avantageusement ovale, particulièrement circulaire. Notamment on trouve une série de cannelures parallèles installées sur la périphérie de l'arbre ou dans ce dernier cas un filetage prévu circonférentiellement.

Dans le cas d'un premier plateau fixe en translation par rapport à l'arbre, éventuellement d'une pièce avec l'axe, la pente douce d'un cran de blocage du plateau mobile sera installée du côté de l'extrémité libre de l'arbre tandis que sa pente abrupte sera installée du côté du premier plateau.

Dans d'autres conditions préférentielles de mise en oeuvre de l'invention, cumulatives avec les précédentes, le puits comprend une ou plusieurs lames flexibles, notamment fixes du côté du second plateau, proximal, et libres du côté distal. Ces lames flexibles sont de préférence prévues environ parallèles à l'axe de l'arbre. Avantageusement cette ou ces lames flexibles comportent à leur surface interne un relief comme un bourrelet aigu ou une arête ou analogue, de préférence prévue à l'extrémité distale ou à proximité de l'extrémité distale. La coopération entre les crantages de l'arbre et les reliefs renforce l'effet cliquet désiré.

Dans encore d'autres conditions préférentielles de mise en oeuvre de l'invention, cumulatives avec les précédentes, l'arbre est muni d'un ou plusieurs évidements pour recevoir une greffe osseuse. Cette lumière peut être notamment une multitude de lumières rapprochées du type nid d'abeilles par exemple. Ces évidements ont une profondeur représentent une partie de la section de l'arbre (la section étant une coupe de l'arbre perpendiculairement à son axe). L'évidement peut représenter par exemple 10 à 90% de la section, de préférence 15 à 80% de la section, notamment de 20 à 75% de la section, particulièrement de 25 à 70% de la section, tout particulièrement de 30 à 65% de la section.

Aussi, ces évidements ont une certaine longueur (dans le sens de l'axe de l'arbre) Il s'agit de la longueur de l'évidement s'il n'y en a qu'un ou la longueur de l'ensemble de la zone évidée lorsqu'il y a plusieurs évidements (dans le cas par exemple d'évidements rapprochés type nid d'abeilles). La longueur de ces évidements pourra être de 1 à 20 mm, de préférence de 2 à 18 mm, notamment de 3 à 15 mm, particulièrement de 4 à 12 mm, tout particulièrement de 5 à 10 mm.

Un système de guidage du déplacement du second plateau le long de l'arbre est avantageusement prévu. A cette fin, l'arbre peut être muni d'une rainure longitudinale coopérant avec une protubérance prévue vers l'intérieur du puits, par exemple au niveau de la plaque.

L'arbre est réalisé avantageusement en polyétheréthercétone (PEEK) ou autre matériau de mêmes propriétés mécaniques et radiotransparents, afin de permettre la visualisation de la greffe lors des contrôles radiographiques. Cependant il peut aussi bien être réalisé en d'autres matériaux implantables polymériques, métalliques, comme en titane allié ou pur.

Le diamètre externe de l'arbre sera par exemple de 2 à 18, de préférence de 4 à 16, notamment de 5 à 15, tout particulièrement de 6 à 14 mm. Pour que l'arbre serve aussi de cale interépineuse (spacer) le diamètre externe de l'arbre sera par exemple de 5 à 20, de préférence de 6 à 20, notamment de 6 à 18, tout particulièrement de 8 à 18 mm.

La longueur l'arbre sera par exemple de 20 à 50, de préférence de 25 à 45, notamment de 25 à 40, tout particulièrement de 30 à 35 mm.

La longueur des plateaux sera par exemple de 1,5 à 7, de préférence de 2 à 6, notamment de 2 à 5, tout particulièrement de 3 à 4 cm.

La largeur maximale des plateaux sera par exemple de 3 à 16, de préférence de 4 à 12, notamment de 5 à 10, tout particulièrement de 6 à 8 mm.

L'épaisseur de la plaque des plateaux sera par exemple de 1 à 8, de préférence de 1,5 à 6, notamment de 1,5 à 4, tout particulièrement de 2 à 3 mm.

La longueur du puits hors plateau (donc en général environ la longueur des lames) sera par exemple de 2 à 14, de préférence de 3 à 12, notamment de 4 à 10, tout particulièrement de 6 à 8 mm.

Les lames pourront être avantageusement au nombre de 1 à 20, de préférence de 4 à 16, notamment de 6 à 12, tout particulièrement de 8 à 10.

Les cannelures pourront être avantageusement au nombre de 4 à 35, de préférence de 6 à 30, notamment de 8 à 25, tout particulièrement de 10 à 20. L'écart entre deux cannelures-crans (creux à creux ou crête à crête) peut aller de 0,2 à 2,5 mm, de préférence de 0,4 à 2 mm, notamment de 0,6 à 1,5 mm.

Les systèmes de pincement d'épineuses objets de la présente invention possèdent de très intéressantes propriétés et qualités. Ils permettent de facilement bloquer leur mouvement relatif vertèbres concernées afin. Les lames élastiques assurent l'irréversibilité du mouvement en verrouillant la position des plateaux et le maintien de la compression sur l'os.

Un important avantage de ce système est la facilité de sa mise en place et sa stabilité dans le temps obtenus grâce au mécanisme à cliquet anti-retour. La mise en place se fait en un seul geste de serrage, une seule main étant nécessaire. De plus il n'y a pas de risque de desserrage d'une vis de blocage dans le temps, ce qui est important car ce type de dispositif est destiné à être implanté pour toute la vie.

Ces qualités sont illustrées ci-après. Elles justifient l'utilisation des ensembles de barres ci-dessus décrits dans la stabilisation de la colonne vertébrale.

C'est pourquoi la présente demande a aussi pour objet une méthode de stabilisation de la colonne vertébrale dans laquelle on installe des systèmes de pincement d'épineuses ci-dessus sur des vertèbres adjacentes et bloque lesdites vertèbres adjacentes. De préférence, en outre, on installe une greffe osseuse dans le ou les évidements, de l'arbre.

Les dispositifs ci-dessus étant pour usage chirurgical, la présente demande a aussi pour objet lesdits dispositifs stériles, notamment conditionnés sous emballage préservant leur stérilité.

Les conditions préférentielles de mise en oeuvre des systèmes de pincement d'épineuses ci-dessus décrites s'appliquent également aux autres objets de l'invention visés ci-dessus, notamment aux procédés et méthodes les mettant en oeuvre et pour leur fabrication.

L'invention sera mieux comprise si l'on se réfère aux dessins annexés sur lesquels
La figure 1 représente une vue en perspective d'un système de pincement d'épineuses selon l'invention dont les pièces sont disjointes.
La figure 2 représente un tel système en position fonctionnelle.
La figure 3 représente une loupe illustrant le système de cliquet, comme la figure 4 qui en est une vue en coupe.
La figure 5 représente le montage d'un système selon l'invention sur des épineuses adjacentes.

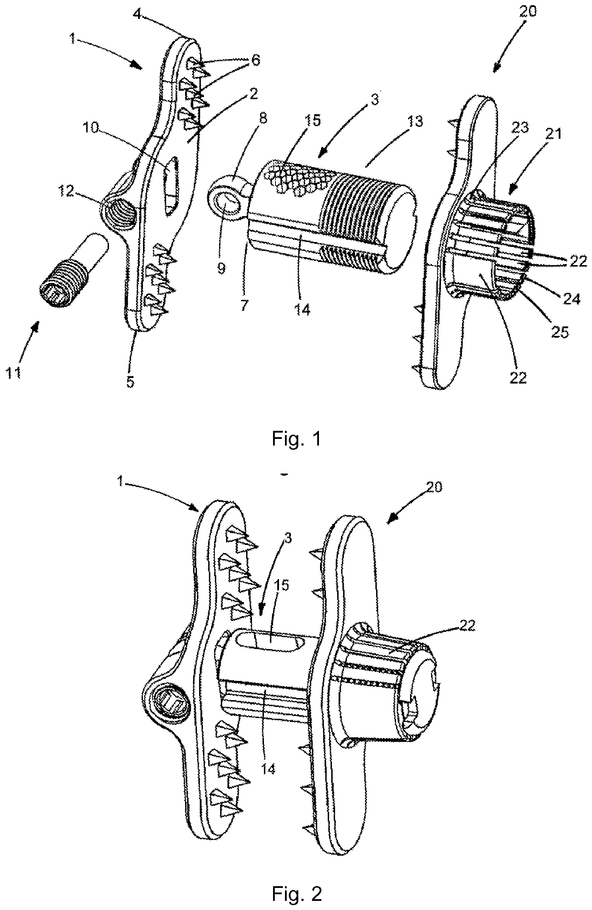

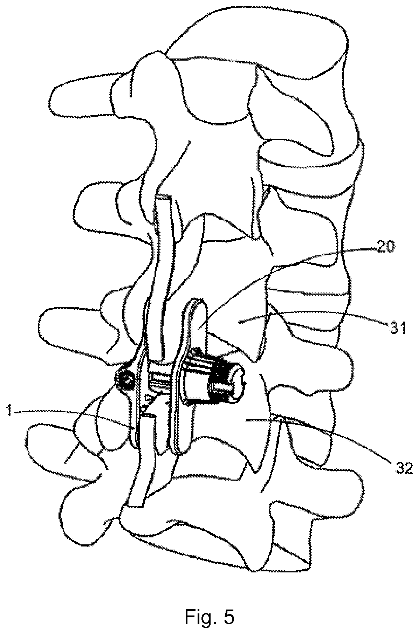

Sur la figure 1, on observe à gauche un premier plateau 1 de forme allongée. La face interne 2 de ce plateau 1, dirigée vers l'arbre 3, est munie vers sa première extrémité 4 et sa seconde extrémité 5 d'aspérités 6 en relief. Ces aspérités 6 pourront se planter dans des épineuses comme on le verra ci-après sur la figure 5. L'arbre 3 est généralement cylindrique et massif. Il comprend à sa première extrémité 7 une ouverture circulaire installée diamétralement par rapport à l'axe de l'arbre et qui est un anneau 8 comportant une lumière 9. Cet anneau 8 peut pénétrer dans une cavité 10 prévue dans le premier plateau. Cette cavité 10 est d'une taille permettant le pivotement de l'arbre (de haut en bas et inversement sur cette figure). Pour solidariser l'arbre 3 avec le premier plateau 1, il est prévu un goujon fileté 11 qui vient se visser dans le canal fileté 12 prévu dans un bossage ménagé sur le premier plateau 1 pour traverser la lumière 9 de l'anneau 8 et bloquer les mouvements en translation de l'arbre 3 vis-à-vis du premier plateau 1, tout en autorisant son pivotement.

Cet arbre 3 comprend à sa partie opposée à l'anneau 8 une série de vingt cannelures 13 circonférentielles parallèles entre elles constituant un crantage. Le système comprend également un second plateau 20, de structure comparable au premier plateau en ce qui concerne le plateau lui-même. Mais au milieu de celui-ci est prévu un puits 21 constitué d'un ensemble de lames 22 fixées par leur base 23 au plateau 20 et dont le sommet 24 est libre. Ainsi, ces lames 22 sont flexibles. Vers l'extrémité 24 des lames 22 est prévue sur chaque lame une arête aigüe 25 dirigée vers l'intérieur du puits. Ces arêtes 25 peuvent coopérer avec les cannelures 13 de l'arbre 3, dans lesquelles elles s'encliquettent pour rendre irréversible le mouvement de rapprochement du premier et du second plateau.

Pour empêcher la rotation relative du second plateau 20 par rapport à l'arbre 3, il est prévu dans l'épaisseur de la paroi de l'arbre 3, deux rainures 14 allongées à section en V, diamétralement opposées, coopérant avec des protubérances de forme complémentaires (non représentées) prévues vers l'intérieur du puits, au niveau du plateau. Quand on pousse le second plateau vers le premier plateau, ce qui peut se faire en s'aidant d'une pince, les lamelles flexibles s'écartent lors du passage d'une cannelure vers l'autre, pour se rapprocher ensuite après passage du relief et se bloquer dans le creux de la cannelure suivante. Comme l'arbre 3 est généralement cylindrique et massif, il ne comprend pas de fente longitudinale séparant l'arbre en deux parties de sorte que ces deux parties puissent être rapprochées l'une de l'autre. Ainsi, la force de maintien produite par les arêtes 25 coopérant avec les cannelures 13 de l'arbre 3 est constante d'un bout à l'autre des cannelures 13, alors que si l'arbre 3 comprenait une fente longitudinale, la force de maintien serait moindre du côté de l'ouverture de la fente, éloignée de la zone de jonction des deux parties.

Dans l'arbre 3, sont prévus des évidements 15 autorisant l'installation de greffes de moelle osseuse. Ces évidements 15 sont en forme de nid d'abeilles ; ils représentent, en coupe perpendiculaire à l'axe, selon l'évidement considéré, une profondeur de 30 à 80% le la section de l'arbre.

Sur la figure 2, le système est monté en vue de son utilisation pour pincer des épineuses.

Sur cette figure, l'arbre 3 comporte un évidement 15 unique de forme oblongue. Il représente environ 60% de la section de l'arbre (coupe perpendiculaire à l'axe de l'arbre). Les rainures 14 n'ont pas une section en V mais en U. L'anneau 8 a été introduit dans la cavité 10 puis solidarisé avec le premier plateau à l'aide du goujon 11 partiellement fileté. Il est muni d'une empreinte hexagonale pour vissage par une clef Allen. Les deux plateaux 1, 20 sont représentés parallèles mais le premier plateau 1, à gauche, est capable de pivoter par exemple pour mieux s'adapter à des épineuses d'épaisseurs différentes et de formes aléatoires.

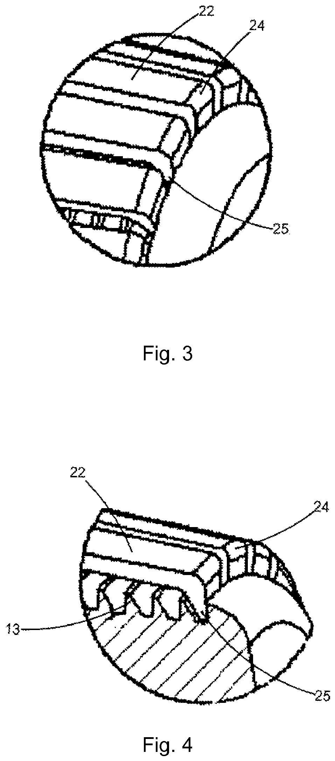

Sur la figure 3, qui est une loupe de la figure 2, on peut mieux observer l'extrémité distale 24 des lames 22 ainsi que leurs arrêtes 25.

Ces éléments sont encore mieux visibles sur la figure 4, sur laquelle on observe les cannelures 13 parallèles entre elles constituant un crantage à section en dents de scie. On peut mieux observer également les arrêtes 25 à l'extrémité 24 des lames 22.

Sur la figure 5, deux vertèbres adjacentes 31 et 32 ont été bloquées par utilisation d'un système selon l'invention, grâce au pincement des épineuses prises en étaux entre le premier plateau 1 et le second plateau 20.

Sur ce modèle, l'arbre 3 a été réalisé en PEEK tandis que les trois autres pièces sont réalisées en titane.

Le diamètre de l'arbre 3 est de 12 mm environ et sa longueur totale, comprenant l'anneau, de 32 mm environ. La longueur des plateaux est de 3 cm environ et leur largeur maximale de 1 cm environ. L'épaisseur de la plaque des plateaux est de 2 mm environ et la longueur du puits hors plateau est de 8 mm environ.

Le diamètre relativement important de l'arbre 3 permet à la fois une bonne coopération arêtes 25/cannelures 13 car le blocage peut s'établir sur toute la circonférence ou presque de l'arbre et en outre l'arbre 3 peut jouer le rôle de cale interépineuse (spacer).

On a également réalisé sur le même principe les 2 systèmes de dimensions et nature suivantes:

| | 2 | 3 |
|---|---|---|
| Matériau utilisé pour l'arbre | peek | T40 |
| Matériau utilisé pour le premier plateau et le second plateau | Ta6v | Ta6v |
| Matériau utilisé pour le goujon | Ta6v | Ta6v |
| Hauteur des plateaux | 2,5 cm | 3cm |
| Largueur maximale des plateaux | 12 mm | 8 mm |
| Epaisseur de la plaque des plateaux | 2,5 mm | 3 mm |
| Longueur du puits hors plateau | 8 mm | 12 mm |
| Longueur totale de l'arbre | 25 mm | 42 mm |

## Revendications

1. Un système de pincement d'épineuses, comprenant un premier (1) et un second plateau (20) installés face à face et un arbre (3) installé environ perpendiculairement aux deux plateaux (1,20) et traversant au moins partiellement l'un d'eux, les plateaux (1,20) comprenant une face interne et une face externe, une surface de la face interne des plateaux (1,20) est munie d'aspérités (6) en relief, en ce que le premier plateau (1) est mobile relativement au second plateau (20), le premier plateau (1) est fixe en translation par rapport à l'arbre (3) **caractérisé en ce que** l'arbre (3) et le second plateau (20) forment un mécanisme à cliquet anti-retour comprenant une série de crans coopérant avec le mécanisme à cliquet anti-retour pour que le rapprochement des plateaux (1,20) soit irréversible sans aide extérieure, en conséquence de quoi des épineuses peuvent être efficacement pincées entre les deux plateaux (1,20).

2. Un système de pincement d'épineuses selon la revendication 1, **caractérisé en ce que** le diamètre externe de l'arbre est de 2 à 18 mm, de préférence de 4 à 18 mm, particulièrement de 4 à 16 mm.

3. Un système de pincement d'épineuses selon la revendication 1, **caractérisé en ce que** le diamètre externe de l'arbre est de 5 à 20 mm.

4. Un système de pincement d'épineuses selon l'une des revendications 1 à 3, **caractérisé en ce que** le premier plateau et l'arbre (3) sont deux pièces distinctes.

5. Un système de pincement d'épineuses selon l'une des revendications 1 à 4, **caractérisé en ce qu'**une des extrémités de l'arbre (3) comprend une lumière circulaire (9) installée diamétralement par rapport à l'axe de l'arbre (3).

6. Un système de pincement d'épineuses selon la revendication 5, **caractérisé en ce que** le premier plateau comprend une cavité (10) pour le passage de l'extrémité de l'arbre (3) et un moyen (11) utilisé comme axe pour la lumière (9) circulaire pour permettre à l'arbre (3) de se mouvoir autour d'un point fixe.

7. Un système de pincement d'épineuses selon l'une des revendications 1 à 6, **caractérisé en ce que** le second plateau (20) comporte un puits (21) permettant le passage de l'arbre (3) et autorisant le rapprochement des plateaux (1,20).

8. Un système de pincement d'épineuses selon l'une des revendications 1 à 7, **caractérisé en ce que** l'arbre est une tige crantée.

9. Un système de pincement d'épineuses selon l'une des revendications 1 à 8, **caractérisé en ce qu'**une série de cannelures (13) parallèles est installée sur la périphérie de l'arbre (3).

10. Un système de pincement d'épineuses selon l'une des revendications 7 à 9, **caractérisé en ce que** le puits (21) comprend une ou plusieurs lames (22) flexibles.

11. Un système de pincement d'épineuses selon la revendication 10, **caractérisé en ce que** les lames (22) flexibles sont fixes du côté proximal et libres du côté distal.

12. Un système de pincement d'épineuses selon l'une des revendications 10 et 11, **caractérisé en ce que** les lames (22) flexibles comportent à leur surface interne un relief (25).

13. Un système de pincement d'épineuses selon l'une des revendications 7 à 12, **caractérisé en ce que** l'arbre (3) est muni d'un ou plusieurs évidements (15) pour recevoir une greffe osseuse. Les évidements pour la greffe sont faits sur l'arbre 3.

14. Un système de pincement d'épineuses selon l'une des revendications 1 à 6, **caractérisé en ce que** l'arbre (3) est muni d'une rainure (14) longitudinale coopérant avec une protubérance prévue vers l'intérieur du puits.

## Patentansprüche

1. System zum Greifen von Dornfortsätzen, mit einer ersten (1) und einer zweiten Platte (20), die einander zugewandt installiert sind, und einem Schaft (3), der in etwa senkrecht zu den zwei Platten (1, 20) installiert ist und zumindest teilweise eine von diesen durchquert, wobei die Platten (1, 20) eine Innenseite und eine Außenseite aufweisen, eine Fläche der Innenseite der Platten (1, 20) mit erhöhten Aufrauhungsmerkmalen (6) versehen ist, die erste Platte (1) relativ zu der zweiten Platte (20) bewegbar ist und die erste Platte (1) relativ zu dem Schaft (3) translatorisch fixiert ist, **dadurch gekennzeichnet, dass** der Schaft (3) und die zweite Platte (20) einen Ratschenmechanismus ausbilden, der eine Reihe von Kerben aufweist, die so mit dem Ratschenmechanismus zusammenwirken, dass die Annäherung der Platten ohne äußere Hilfe irreversibel ist, wodurch die Dornfortsätze wirksam zwischen den zwei Platten (1, 20) gegriffen werden können.

2. System zum Greifen von Dornfortsätzen nach Anspruch 1, **dadurch gekennzeichnet, dass** der Außendurchmesser des Schafts 2 bis 18 mm, bevorzugt 4 bis 18 mm und insbesondere 4 bis 16 mm ist.

3. System zum Greifen von Dornfortsätzen nach Anspruch 1, **dadurch gekennzeichnet, dass** der Außendurchmesser des Schafts 5 bis 20 mm ist.

4. System zum Greifen von Dornfortsätzen nach einem der Ansprüche 1 bis 3, **dadurch gekennzeichnet, dass** die erste Platte und der Schaft (3) zwei getrennte Teile sind.

5. System zum Greifen von Dornfortsätzen nach einem der Ansprüche 1 bis 4, **dadurch gekennzeichnet, dass** eines der Enden des Schafts (3) eine kreisförmige Öffnung (9) aufweist, die relativ zu der Achse des Schafts (3) diametrisch angeordnet ist.

6. System zum Greifen von Dornfortsätzen nach Anspruch 5, **dadurch gekennzeichnet, dass** die erste Platte eine Aussparung (10) für den Durchgang des Endes von dem Schaft (3) und ein Mittel (11) aufweist, das als Achse für die kreisförmige Öffnung (9) verwendet wird, um es dem Schaft (3) zu ermöglichen, sich um einen Fixpunkt zu bewegen.

7. System zum Greifen von Dornfortsätzen nach einem der Ansprüche 1 bis 6, **dadurch gekennzeichnet, dass** die zweite Platte (20) eine Vertiefung (21) aufweist, die ermöglicht, dass der Schaft (3) hindurchgeht und welche die Annäherung der Platten (1, 20) zulässt.

8. System zum Greifen von Dornfortsätzen nach einem der Ansprüche 1 bis 7, **dadurch gekennzeichnet, dass** der Schaft ein eingekerbter Stab ist.

9. System zum Greifen von Dornfortsätzen nach einem der Ansprüche 1 bis 8, **dadurch gekennzeichnet, dass** bei dem Umfang des Schafts (3) eine Reihe von parallelen Nuten (13) ausgebildet ist.

10. System zum Greifen von Dornfortsätzen nach einem der Ansprüche 7 bis 9, **dadurch gekennzeichnet, dass** die Vertiefung (21) eine oder mehrere flexible Lamellen (22) aufweist.

11. System zum Greifen von Dornfortsätzen nach Anspruch 10, **dadurch gekennzeichnet, dass** die flexiblen Lamellen (22) an der proximalen Seite feststehend und an der distalen Seite flexibel sind.

12. System zum Greifen von Dornfortsätzen nach einem der Ansprüche 10 und 11, **dadurch gekennzeichnet, dass** die flexiblen Lamellen (22) auf ihren inneren Flächen eine Erhöhung (25) aufweisen.

13. System zum Greifen von Dornfortsätzen nach einem der Ansprüche 7 bis 12, **dadurch gekennzeichnet, dass** der Schaft (3) mit einer oder mehreren Aussparungen (15) versehen ist, um ein Knochentransplantat aufzunehmen, wobei die Aussparungen für das Transplantat an dem Schaft (3) ausgebildet sind.

14. System zum Greifen von Dornfortsätzen nach einem der Ansprüche 1 bis 6, **dadurch gekennzeichnet, dass** der Schaft mit einem länglichen Kanal (14) versehen ist, der mit einer Protuberanz zusammenwirkt, die in Richtung der Innenseite der Vertiefung vorgesehen ist.

## Claims

1. A system for gripping spinous processes, comprising a first plate (1) and a second plate (20), which are installed face to face, and a shaft (3), which is installed approximately perpendicularly with respect to the two plates (1, 20) and passes at least partially through one of them, the plates (1, 20) comprising an inner face and an outer face, wherein a surface of the inner face of the plates (1, 20) is provided with raised roughening features (6), wherein the first plate (1) is movable relative to the second plate (20), wherein the first plate (1) is fixed in translation with respect to the shaft (3), **characterized in that** the shaft (3) and the second plate (20) form a non-return pawl mechanism comprising a series of notches that cooperate with the non-return pawl mechanism such that the movement of the plates (1, 20) toward each other is irreversible without external aid, whereby spinous processes can be effectively gripped between the two plates (1, 20).

2. The system for gripping spinous processes as claimed in claim 1, **characterized in that** the external diameter of the shaft is 2 to 18 mm, preferably 4 to 18 mm, particularly 4 to 16 mm.

3. The system for gripping spinous processes as claimed in claim 1, **characterized in that** the external diameter of the shaft is 5 to 20 mm.

4. The system for gripping spinous processes as claimed in one of claims 1 through 3, **characterized in that** the first plate and the shaft (3) are two separate components.

5. The system for gripping spinous processes as claimed in one of claims 1 through 4, **characterized in that** one of the ends of the shaft (3) comprises a circular hole (9) arranged diametrically with respect to the axis of the shaft (3).

6. The system for gripping spinous processes as claimed in claim 5, **characterized in that** the first plate comprises a cavity (10), for passage of the end of the shaft (3), and a means (11) used as an axle for the circular hole (9) in order to allow the shaft (3) to move about a fixed point.

7. The system for gripping spinous processes as claimed in one of claims 1 through 6, **characterized in that** the second plate (20) has a well (21) permitting passage of the shaft (3) and allowing the plates (1, 20) to be moved toward each other.

8. The system for gripping spinous processes as claimed in one of claims 1 through 7, **characterized in that** the shaft is a notched rod.

9. The system for gripping spinous processes as claimed in one of claims 1 through 8, **characterized in that** a series of parallel grooves (13) is arranged on the periphery of the shaft (3).

10. The system for gripping spinous processes as claimed in one of claims 7 through 9, **characterized in that** the well (21) comprises one or more flexible blades (22).

11. The system for gripping spinous processes as claimed in one of claims 1 through 8, **characterized in that** the flexible blades (22) are fixed at the proximal end and free at the distal end.

12. The system for gripping spinous processes as claimed in either of claims 10 and 11, **characterized in that** the flexible blades (22) have a protrusion (25) on their inner surface.

13. The system for gripping spinous processes as claimed in one of claims 7 through 12, **characterized in that** the shaft (3) is provided with one or more recesses (15) for receiving a bone graft. The recesses for the graft are formed on the shaft 3.

14. The system for gripping spinous processes as claimed in one of claims 1 through 6, **characterized in that** the shaft (3) is provided with a longitudinal channel (14) cooperating with a protuberance provided toward the inside of the well.
